# NEW EUROPEAN PATENT SPECIFICATION

(11) **EP 3 707 517 B2**
(45) Date of publication and mention of the opposition decision: **03.06.2026**
(45) Mention of the grant of the patent: 28.06.2023
(21) Application number: 18807557.6
(22) Date of filing: 07.11.2018
(51) Int. Cl.: G01N 35/00, G07F 11/62, A61B 10/00

(54) **STATIC AUTOMATIC READING SYSTEM OF HISTOLOGY CASSETTE CODES**
STATISCHES AUTOMATISCHES LESESYSTEM VON HISTOLOGIE-KASSETTENCODES
SYSTÈME DE LECTURE AUTOMATIQUE STATIQUE DES CODES D'HISTOLOGIE CASSETTE

(30) Priority: 09.11.2017 IT 201700128188
(43) Date of publication of application: 16.09.2020
(73) Proprietor: Mecatron Automazione S.r.l., 64010 Colonnella (TE) (IT)
(72) Inventor: CINTI, Mario, 65100 Ascoli Piceno (IT)
(74) Representative: Dall'Olio, Christian
(86) International application number: PCT/EP2018/025283
(87) International publication number: WO 2019/091597

(56) References cited:
- US-A1- 2013 306 729
- US-A1- 2014 330 427
- US-A1- 2017 293 719
- NOWAKOWSKI S E ET AL: "A flexible system to capture sample vials in a storage box - the box vial scanner", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 1718 - 1721, XP031638658, ISBN: 978-1-4244-3296-7

## Description

### Field of the invention

This invention concerns the field of histology, in particular the sector related to histology cassettes and more specifically to a static reading system of codes located on the oblique fronts of said histology cassettes.

### Background of the invention

As per with the current state of the art of histology cassettes, it is known that said cassettes are marked on their oblique side at 45°, rarely at 30° and 90° (perpendicular rather than inclined side) with 1D-2D or alphanumeric codes.

The cassettes are marked to identify them, trace them during work processing and, during the final stage, archive them in an organised manner in specific cabinets. Various technologies can be used to mark the cassettes, such as, inkjet jet printers, thermal transfer, laser or ribbon with indelible ink.

It is known that, to read the codes on the cassettes, these are placed inside trays, normally marked on coordinates with letters and numbers to facilitate identification of each individual cassette. The cassettes are housed in slots along the entire length of the tray, forming "n" side-by-side columns.

Each column is as wide as the histology cassettes and contains tabs separating the cassettes, placed one after the other, ensuring each cassette is kept in position, normally vertically, with the front containing the code facing upwards.

In the present state of the art, document WO2015040320 is known and discloses a method for reading cassette codes, consisting in the positioning of a reader on the Cartesian axes, which, by moving along the X and Y coordinates, with a fixed Z coordinate, determine the reading coordinates of each position and therefore a matrix of reading points (heights) that identify the position of the cassettes inside the container.

Said known system has the disadvantage of being dynamic, that is, being equipped with a moving reader on Cartesian axes, involving lengthy data acquisition times. Frequently, this system does not identify cassettes that do not have perfectly written labels and significant mistakes can be made.

At the state of the art, in the sector related to histology cassettes, the following documents are known:
- US 2017/293719 A1 that discloses a device and a method which can be used in sample processing processes and that simultaneously and rapidly identify, read and transcribe several one-dimensional or two-dimensional computer codes;
- US 2014/330427 A1 that discloses a computerized and automated system for ordering biological sampling blocks;
- Nowakowski S E et al that discloses a device for capturing the location of sample vials which captures, with a single scan, the box identifier, the vial identifier and the location of each vial within a storage box;
- US 2013/306729 A1 that discloses a device for capturing an image of a test tube rack comprising a plurality of test tubes each having an individual barcode thereon, said device comprising:
   - an enclosure comprising a transparent window which, in use, is adjacent to the test tube rack to be imaged;
   - at least one camera;
   - a plurality of light sources;
   - at least one light blocking element, placed for blocking the light at least partially.

None of the known systems provides a simultaneous reading of the identification code of the cassettes contained in a tray as well as the identification code of the same tray. Therefore, none of the known systems allows a high level of traceability of the histological cassettes as well as a reduced possibility of committing acquisition errors.

With the known systems it is not possible to reach a univocal association of cassette/tray data and therefore obtain necessary data for a correct storage of a histological sample.

### Disclosure of the Invention

The object of this invention is to create a static reading system of histology cassette identification codes, that is, with a fixed reader and fixed trays. Another object of this invention is to create a reading system of histology cassette identification codes that is easier to create and use than the existing state of the art.

Another object of this invention is to create a system that allows the acquisition of data in a much faster manner by simultaneously reading all cassettes contained in a specific tray, in addition to the code of the same tray, enabling unequivocal association.

Another object of this invention is to create a system that allows the acquisition of data with a very small possibility of errors.

Lastly, the object of this invention is to create a system that enables an elevated level of cassette traceability.

These and other objects are attained with this invention, which is defined in the claims.

Additional characteristics and advantages of the invention will become more apparent from the description of a preferred, but not exclusive, form of implementation of the system forming the subject matter of this patent application, illustrated by way of indication, but not limited to, the drawing units represented by:

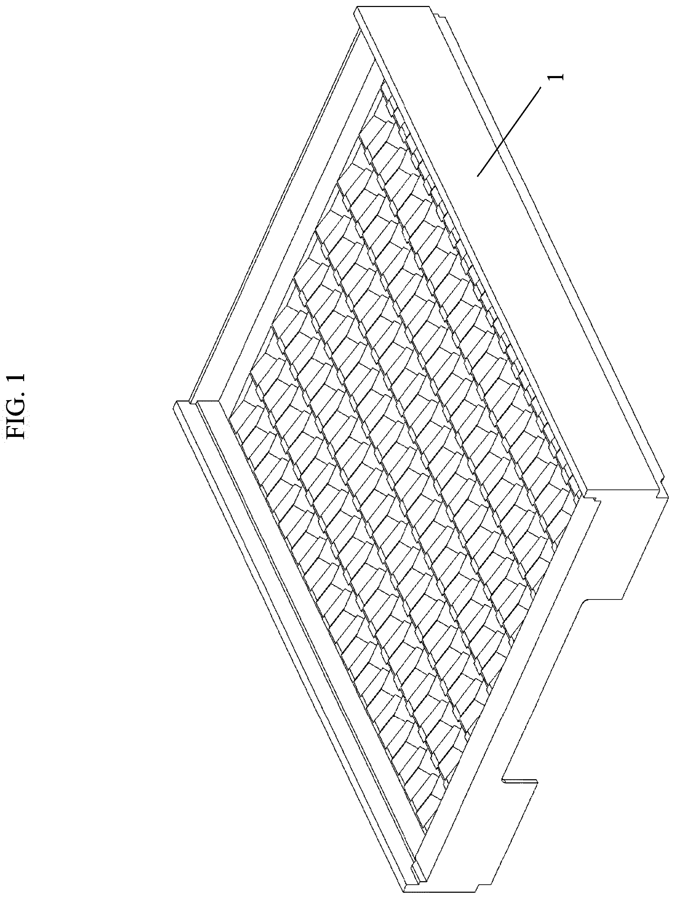

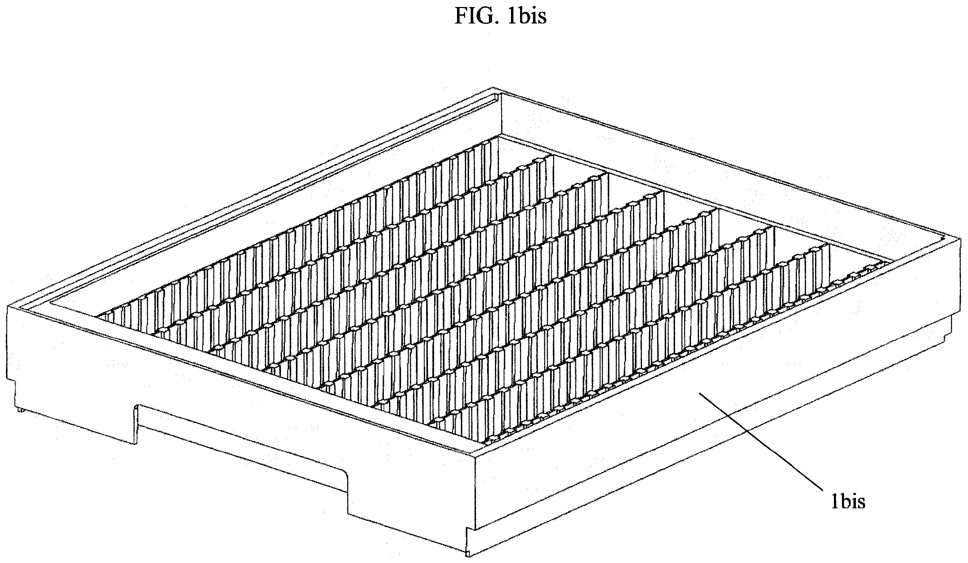

- Fig. 1 shows a three-dimensional view of a tray (1) housing cassettes (2) slanting at an angle;
- Fig 1 bis shows a three-dimensional view of a tray (1bis) housing cassettes (2) vertically;

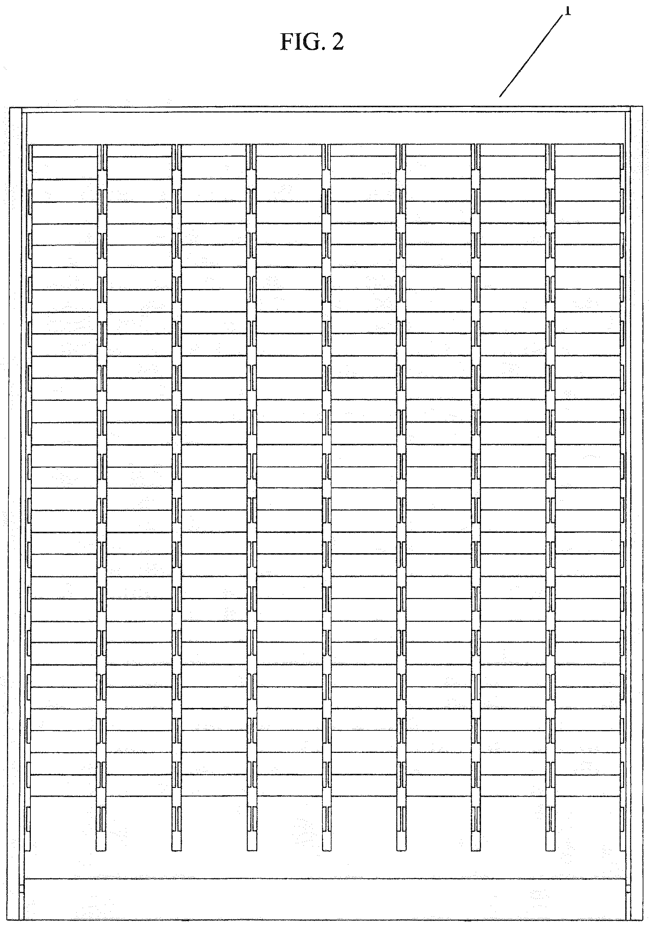

- Fig. 2 shows the tray (1) from above;

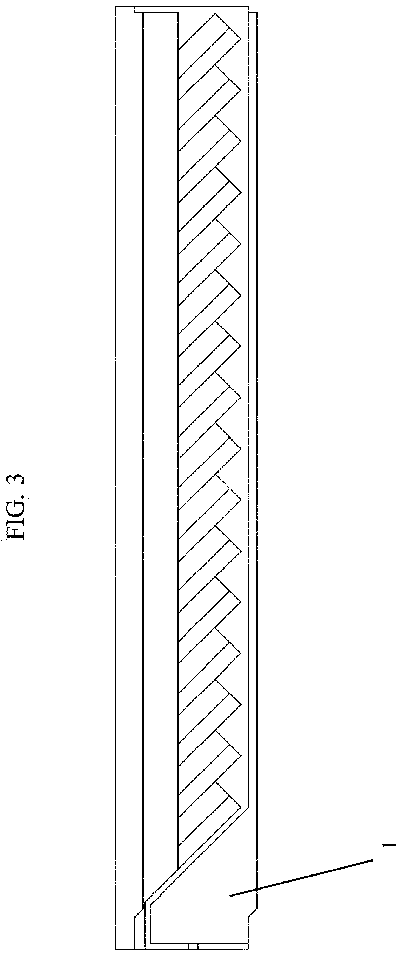

- Fig. 3 shows a sectional view of the tray (1);
- Fig. 4 shows a lateral view of the tray (1);

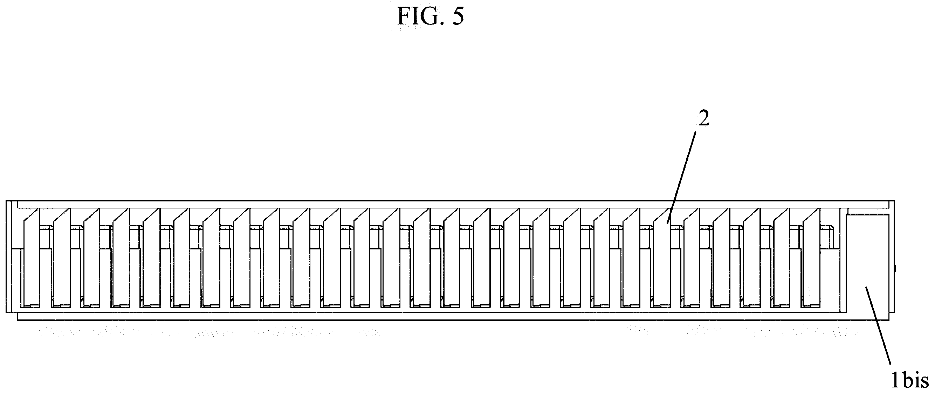

- Fig. 5 shows a sectioned view of the tray (1bis) in which cassettes (2) are housed vertically;

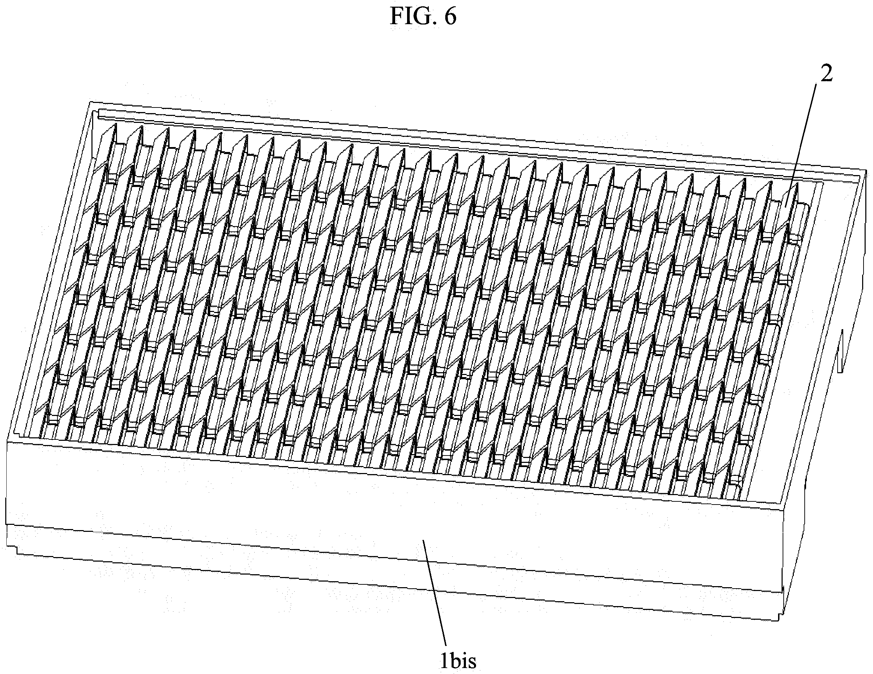

- Fig. 6 shows a three-dimensional view of the tray (1bis) in which cassettes (2) are housed vertically;
- Fig. 7 shows a three-dimensional view of a reading sensor (4) positioned with the horizontal reading beam centring the tray (1) placed vertically;
- Fig. 8 shows a three-dimensional view of a reading sensor (4) positioned with the vertical reading beam centring the tray (1) placed horizontally;
- Fig. 9 represents a three-dimensional view of a reading sensor (4) which reads indirectly by means of angular mirrors or prisms (5);
- Fig. 10 shows a three-dimensional view of a one-row container (3) enabling reading of the identification code on three sides, equipped with springs (7) containing the cassette (2).

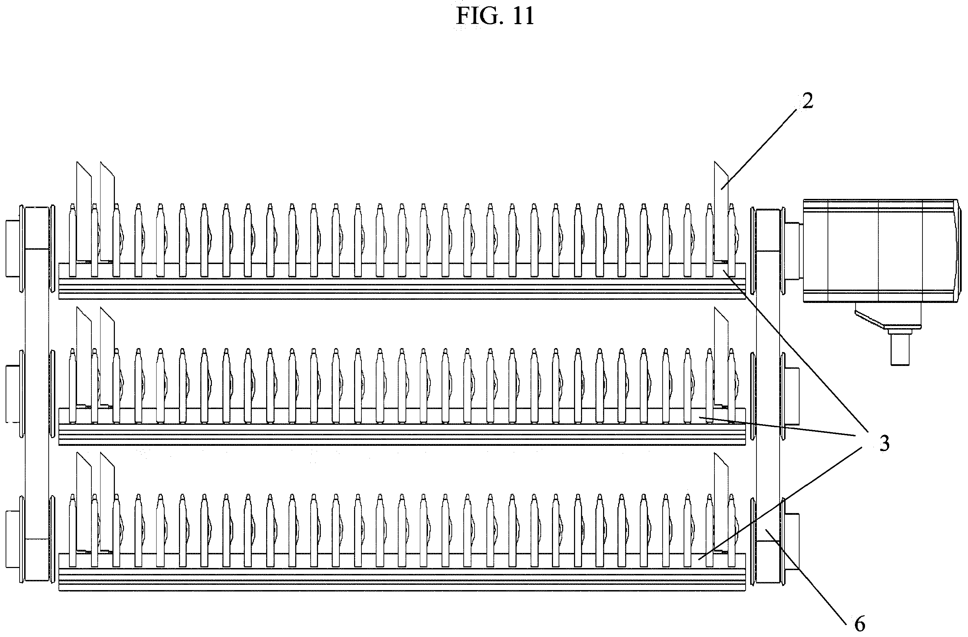

- Fig. 11 shows from above three one-row containers (3) held together by a transmission belt (6) and shows the cassettes (2) rotated clockwise by 90°;

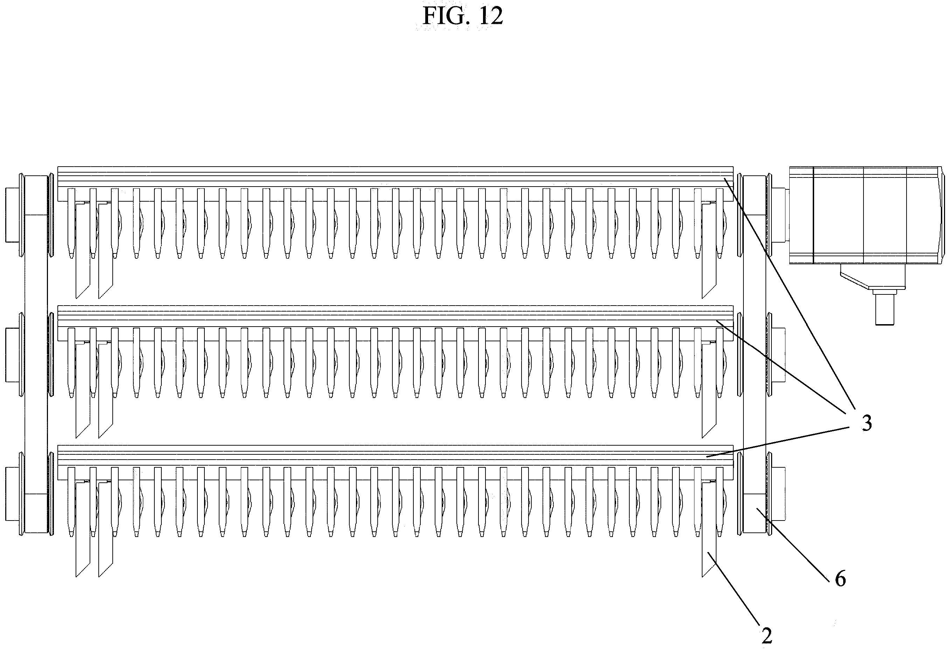

- Fig. 12 shows from above three containers (3) showing the cassettes (2) rotated anti-clockwise by 90°;

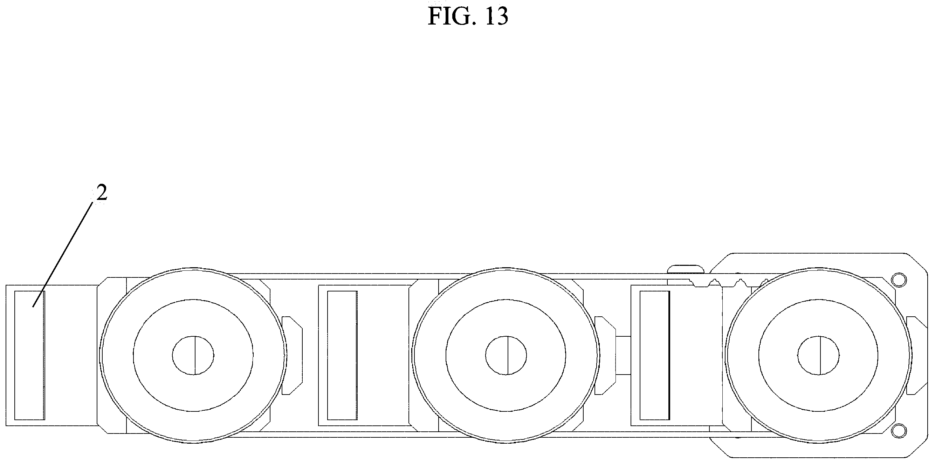

- Fig. 13 shows a lateral view of the three containers (3) in Fig. 12;

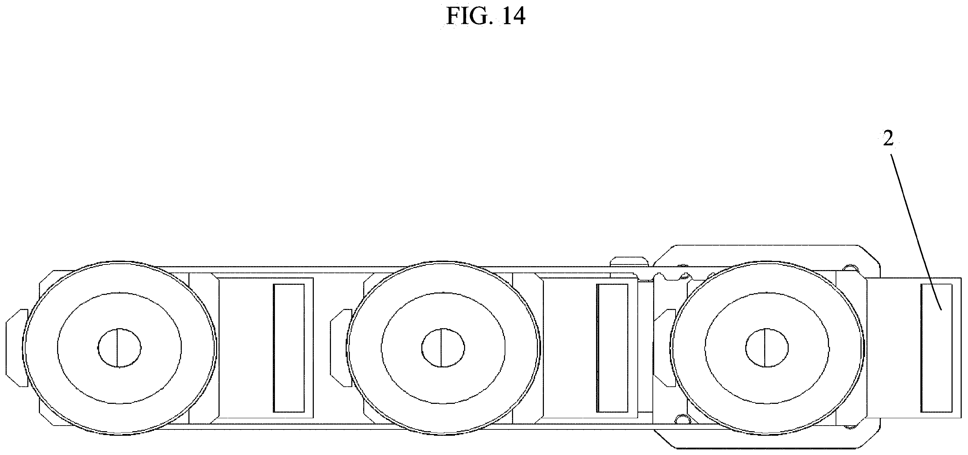

- Fig. 14 shows a lateral view of the three containers (3) in Fig. 11;

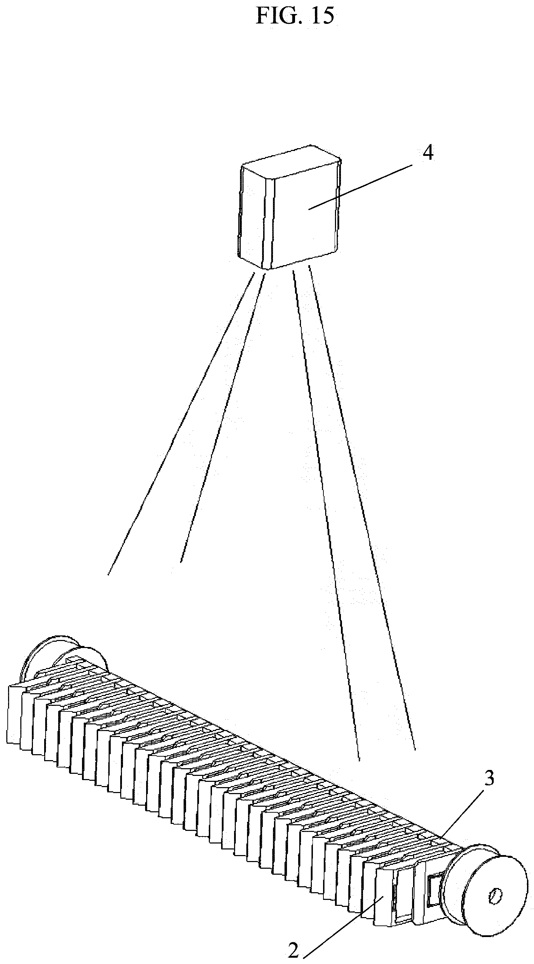

- Fig. 15 shows a three-dimensional view of the reading sensor (4) reading the one-row container (3) with the cassettes (2) rotated anti-clockwise by 90°;

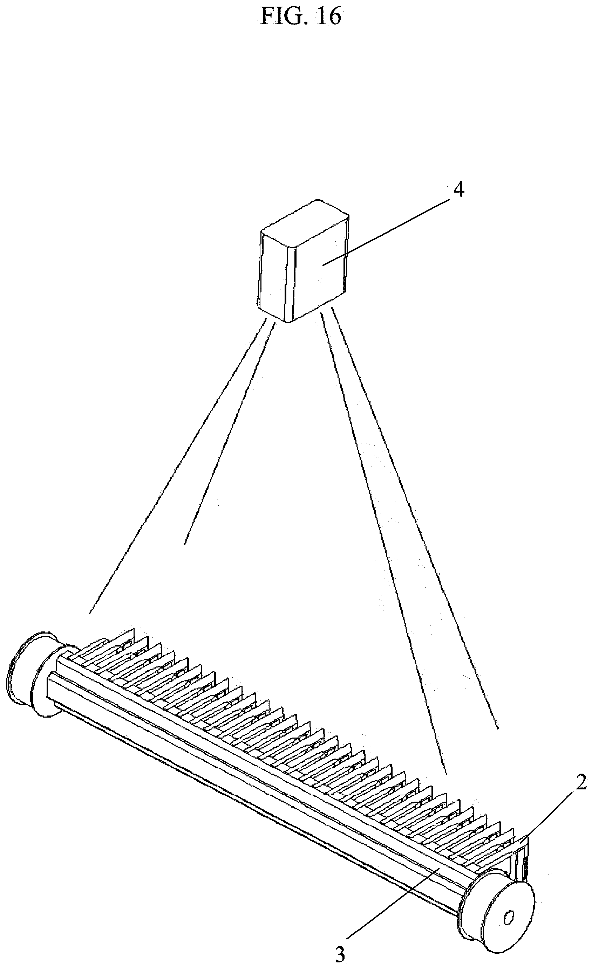

- Fig. 16 shows a three-dimensional view of the reading sensor (4) reading the one-row container (3) with the cassettes (2) rotated clockwise by 90°;

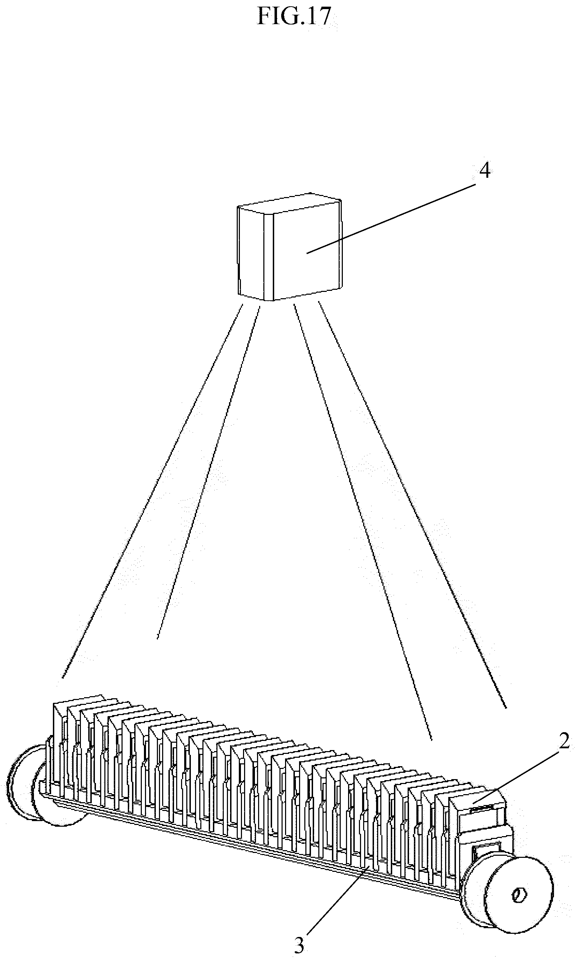

- Fig. 17 shows a three-dimensional view of the reading sensor (4) reading the one-row container (3) with the cassettes (2) positioned vertically.

### Detailed Description of the Invention

In accordance with a preferred - but not limiting - form of implementation, this invention concerns a static reading system of histology cassette codes (2) located on the oblique front of the same cassettes. A static reading system is a system that does not require any electrical/mechanical organ of movement.

The reading system includes:
- fixed trays (1);
- cassettes (2) housed in tray compartments (1) or alternatively in containers (3);
- a fixed reading sensor (4).

Said reading system is able to statically read one or more rows of cassettes (2) arranged in an orderly manner in tray compartments (1), that is, without a reading sensor (4) moving along one or more axes.

Said sensor (4) is to be positioned in a fixed manner above the tray (1) holding the cassettes (2) to be read and, by centring the complete image of the tray (1), defines, by means of software, the reading points and positions of the various cassettes (2).

The reading sensor (4), also understood to mean a scanner or multiple image capturing element, captures, without moving, the image of the entire tray area (1) and its content and generates, by means of software, small areas containing individual cassettes and a grid of positions of the cassettes (2) in the tray (1) based on alphanumeric coordinates.

Each square of the grid containing a cassette (2) is assigned a position, to which the data of the cassette (2) being read is associated. As a result, the reading grid is defined by software processing without the sensor or cassette row (2) or the tray (1) moving.

In this invention, the tray identification code (1) is read by the same sensor (4) at the same time as the cassettes (2) in the tray, while in known systems, the identification code of the tray is read by a handheld scan-read device connected to a computer before being placed in the reading compartment, and then reread when the tray is placed in the compartment and the cassette data is read. It follows that, with regard to traceability, the system forming the subject matter of this patent application is much safer and more immediate.

In the known system, the presence of a cassette is detected by reading each tray compartment, each time moving an optical model, step by step or continuously, following a variety of trajectories, acquiring images in accordance with X and Y coordinate values previously assigned by software; for each assigned position value, the optical model emits a beam of light to capture the image and position.

The development software connected to the system referred to in this invention statically:
- creates and identifies the reading grid;
- identifies the position of a cassette (2);
- identifies whether a cassette (2) is present in a square;
- provides information as to whether the code has been read or whether it could not be read;
- memorises identification by taking a photograph of the image.

The software, in accordance with the image read (cassette read correctly, unrecognised cassette present or cassette not present), unambiguously displays on the screen whether the cassette read is positioned in the compartment by employing different colours on the tray (1) grid generated by the software.

The software is able to recreate and display various reading anomalies on the screen, on the tray (1) grid generated by the software, presenting each anomaly with a different colour.

Said software recreates an on-screen database and provides all the information contained in the cassette (2) code read for each position.

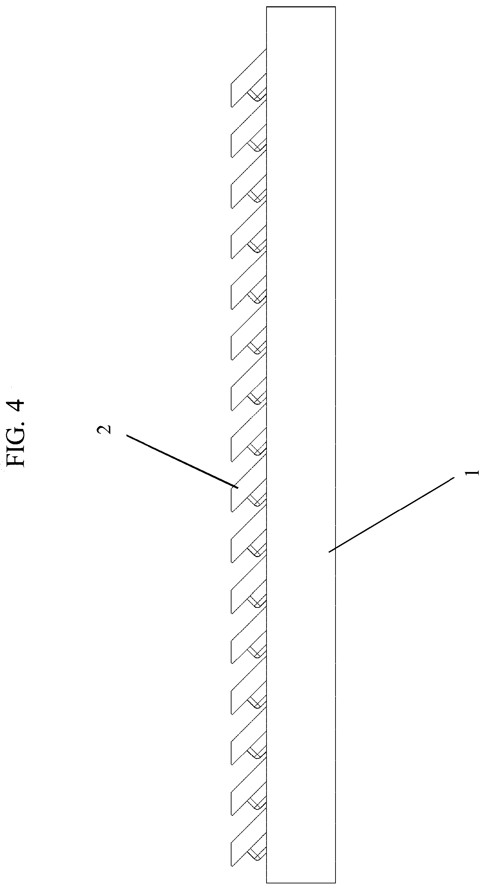

In the system forming the subject matter of this patent application, the cassettes (2) are placed in the tray (1) at an inclined angle, that is, at an angle equal to the angle of the titled side of the cassette on which the identification codes are written, in order to form a reading plane of the codes printed on the cassettes (2) which is perfectly horizontal and parallel to the bottom of the tray (1) and perpendicular to the tray area reader (1) (Fig. 4).

Therefore, the cassettes (2) can be placed vertically in the tray and the sensor (4) will read the slanting front, alternatively, the cassettes can be placed inclined at an angle corresponding to the tilt angle of the front of the cassettes (2).

The number of cassettes (2) that can be read depends on how high the sensor (4) is positioned, since the higher it is positioned, the greater the centring area becomes. Reader pixel definition guaranteeing the definition of reading of the full tray (1) is also a determining factor.

Various trays (1) can easily be composed (coupled) in order to obtain greater cassette capacities (2), just as multiple tray (1) loading and unloading systems can be foreseen.

In comparison to known systems, both the tray (1) and the sensor (4) are fixed.

Individual trays (1) are manually placed inside the reading compartment and centred using guides and frames.

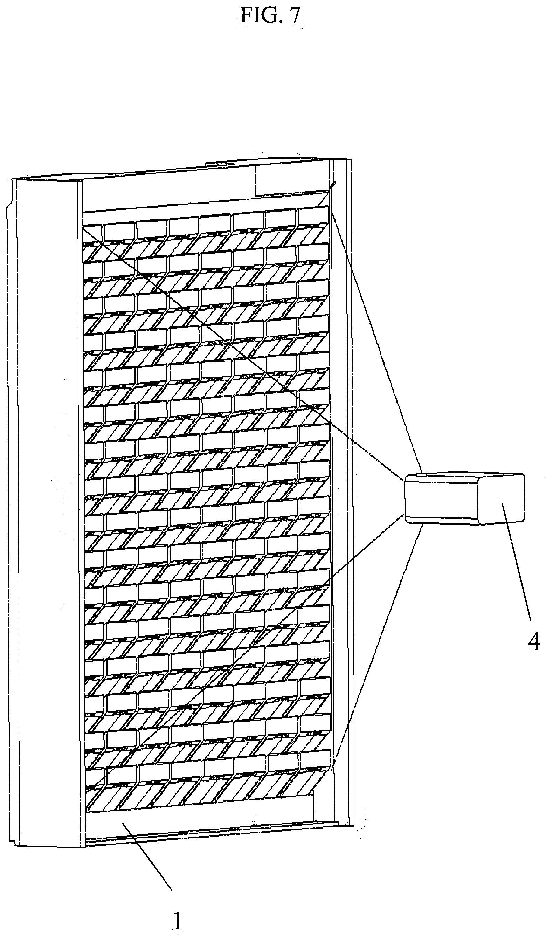

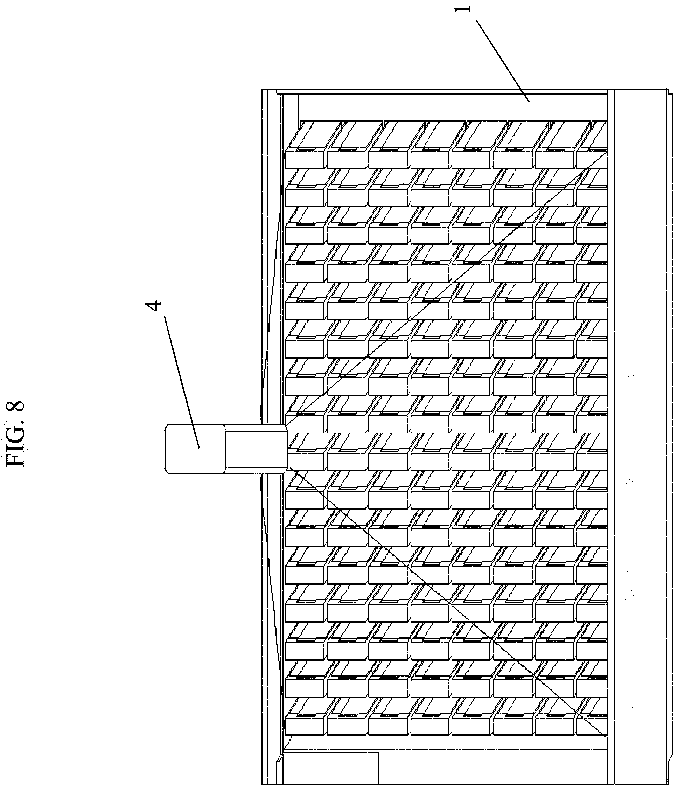

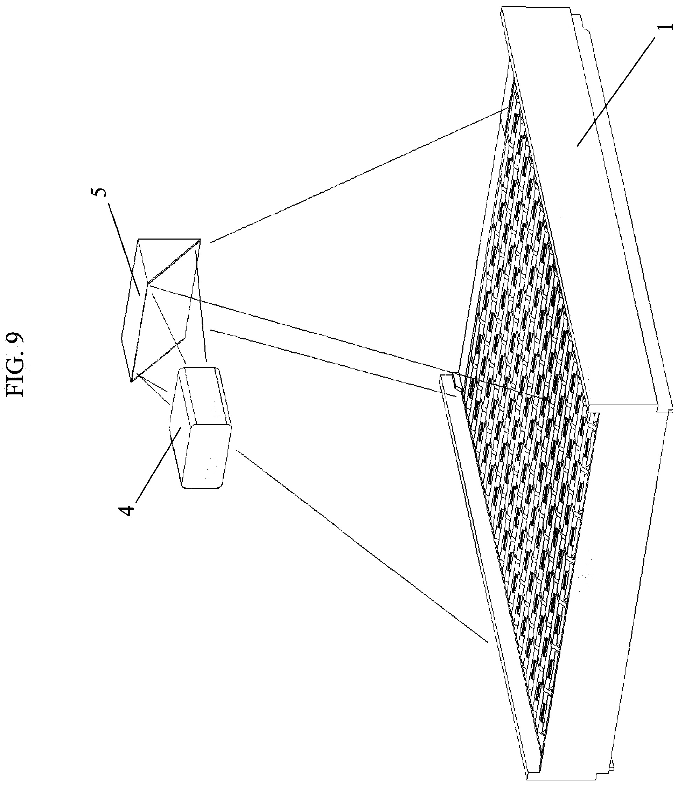

The scanner can be positioned with the reading beam horizontal, centring the tray placed vertically (Fig. 7), or with the reading beam vertical, centring the tray placed horizontally (Fig. 8), or can read indirectly by means of angular mirrors or prisms (5) (Fig. 9).

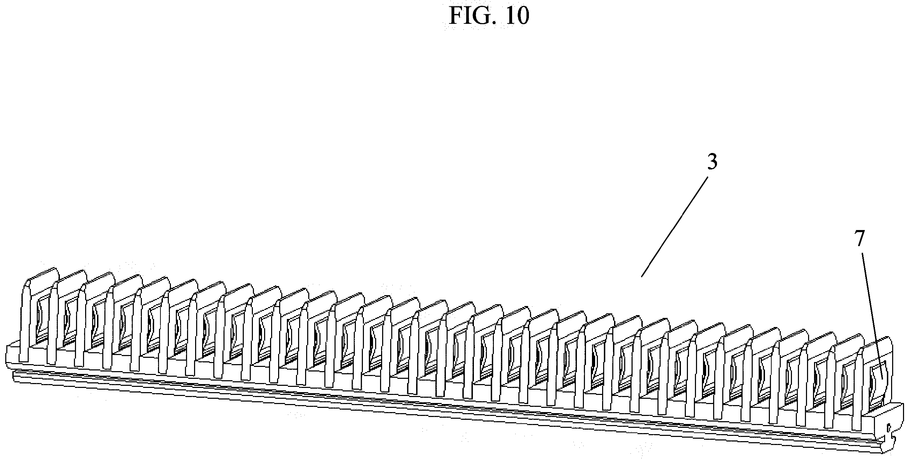

The static reading system forming the subject matter of this patent application can also read cassettes (2) printed on three sides using specific containers (3), which, by means of springs (7), contain a row of cassettes each (Fig. 10); several containers (3) are placed side by side and, if necessary, placed in a tray. Said one-row containers (3) are placed side by side at a such distance to allow rotation of the same clockwise or anti-clockwise by 90° to enable reading of the two lateral sides of the histology cassettes (2); furthermore, the various containers (3) are held together by a belt or another transmission organ (6) guaranteeing their synchronous movement during rotation.

Said static reading system is also able to automatically read alphanumeric codes with previously stored character fonts using the same sensor.

The materials and sizes of the invention as described above, illustrated in the accompanying drawings and claimed hereunder, may be of any variation in accordance with requirements. In addition, all details can be replaced with other technically equivalent details without departing from the scope of the invention as claimed.

## Claims

1. An automatic reading system of codes on the oblique front of cassettes comprising:
- a tray (1);
- cassettes (2) housed in compartments of the tray (1) or in containers (3) placed side by side in the tray (1);
- a reading sensor (4);
the tray (1) being fixed with respect to the reading sensor (4) and the reading sensor (4) positioned in a fixed manner with respect to the tray (1);
wherein said reading sensor (4)
- reads the whole area of the tray (1) and its contents;
- simultaneously reads the identification code of the tray (1) and the identification codes of the cassettes (2) in the tray (1);
- defines, by means of a reading software, small areas containing individual cassettes (2) and a grid of positions of the cassettes (2) in the tray (1) based on alphanumeric coordinates.

2. The reading system according to claim 1, **characterised in that** the cassettes (2) are placed in the tray (1) at an inclined angle, that is, at an angle equal to the angle of the titled side of the cassette on which the identification codes are written, in order to form a reading plane of the codes printed on the cassettes (2) which is horizontal and parallel to the bottom of the tray (1) and perpendicular to the tray area reader (1).

3. The reading system according to claim 1, **characterised in that** the cassettes (2) can be placed entirely vertically and the sensor (4) will read the slanting front, alternatively, the cassettes can be placed inclined at an angle corresponding to the tilt angle of the front of the cassettes (2).

4. The reading system according to claim 1, **characterised in that** the sensor (4) can read the whole tray (1) by means of angular prisms or mirrors (5).

5. The reading system according to claim 1, **characterised in that** each of the containers (3) is a one-row container (3), each of the one-row container (3) is rotatable clockwise and anti-clockwise by 90° allowing three side of the cassettes (2) to be visible and read by the reading sensor (4) and **in that** several one-row containers (3) are placed side by side at a such distance to allow clockwise or anti-clockwise rotation by 90° and are held together by a belt or another transmission organ (6) guaranteeing their synchronous movement during rotation.

## Patentansprüche

1. Automatisches Lesesystem für Codes auf der abgeschrägten Vorderseite von Einbettkassetten, umfassend:
- ein Tray (1);
- Kassetten (2), die in Fächern des Trays (1) oder in im Tray (1) nebeneinander eingelegten Behältern (3) untergebracht sind;
- einen Lesesensor (4);
wobei das Tray (1) in Bezug auf den Lesesensor (4) feststehend ist und der Lesesensor (4) in Bezug auf das Tray (1) in einer festen Position angeordnet ist;
wobei der Lesesensor (4)
- den gesamten Bereich des Trays (1) und dessen Inhalt liest;
- gleichzeitig den Identifikationscode des Trays (1) und die Identifikationscodes der Kassetten (2) in dem Tray (1) liest;
- mittels einer Lesesoftware kleine Bereiche definiert, die einzelne Kassetten (2) enthalten, und ein Gitter von Positionen der Kassetten (2) in dem Tray (1) auf Grundlage alphanumerischer Koordinaten definiert.

2. Lesesystem nach Anspruch 1, **dadurch gekennzeichnet, dass** die Kassetten (2) in dem Tray (1) in einem geneigten Winkel eingelegt sind, das heißt, in einem Winkel, der gleich dem Winkel der abgeschrägten Seite der Kassette ist, die mit den Identifikationscodes beschriftet ist, um eine Leseebene zum Lesen der auf den Kassetten (2) aufgedruckten Codes zu bilden, die horizontal und parallel zu dem Boden des Trays (1) und orthogonal zu dem Leser des Bereichs des Trays (1) ist.

3. Lesesystem nach Anspruch 1, **dadurch gekennzeichnet, dass** die Kassetten (2) vollständig vertikal angeordnet werden können und der Sensor (4) die abgeschrägte Vorderseite liest, oder die Kassetten alternativ dazu geneigt angeordnet werden können, mit einem Winkel, der dem Neigungswinkel der Vorderseite der Kassetten (2) entspricht.

4. Lesesystem nach Anspruch 1, **dadurch gekennzeichnet, dass** der Sensor (4) das gesamte Tray (1) mittels Winkelprismen oder Winkelspiegeln (5) lesen kann.

5. Lesesystem nach Anspruch 1, **dadurch gekennzeichnet, dass** jeder der Behälter (3) ein einreihiger Behälter (3) ist, wobei der einreihige Behälter (3) im und gegen den Uhrzeigersinn um 90° drehbar ist, um zu ermöglichen, dass drei Seiten der Kassetten (2) sichtbar und lesbar für den Lesesensor (4) sind, und dass mehrere einreihige Behälter (3) nebeneinander in einem solchen Abstand voneinander angeordnet sind, der ihre Drehung um 90° im oder gegen den Uhrzeigersinn ermöglicht, und dass sie durch einen Riemen oder ein anderes Bewegungsübertragungsorgan (6) zusammengehalten werden, der/das ihre synchrone Bewegung während der Drehung gewährleistet.

## Revendications

1. Un système de lecture automatique de codes sur le front oblique de cassettes comprenant :
- un plateau (1) ;
- des cassettes (2) logées dans des compartiments du plateau (1) ou dans des récipients (3) placés côte à côte dans le plateau (1) ;
- un capteur de lecture (4) ;
le plateau (1) étant fixe par rapport au capteur de lecture (4) et le capteur de lecture (4) étant positionné de façon fixe par rapport au plateau (1) ;
dans lequel ledit capteur de lecture (4)
- lit toute la zone du plateau (1) et son contenu ;
- lit simultanément le code d'identification du plateau (1) et les codes d'identification des cassettes (2) dans le plateau (1) ;
- définit, par le biais d'un logiciel de lecture, de petites zones contenant des cassettes (2) individuelles et une grille de positions des cassettes (2) dans le plateau (1) selon des coordonnées alphanumériques.

2. Le système de lecture selon la revendication 1, **caractérisé en ce que** les cassettes (2) sont placées dans le plateau (1) à un angle incliné, à savoir, à un angle égal à l'angle du côté incliné de la cassette sur lequel sont écrits les codes d'identification, de manière à former un plan de lecture des codes imprimés sur les cassettes (2) qui est horizontal et parallèle au fond du plateau (1) et perpendiculaire au lecteur de la zone du plateau (1).

3. Le système de lecture selon la revendication 1, **caractérisé en ce que** les cassettes (2) peuvent être placées complètement à la verticale et le capteur (4) lira le front incliné, ou bien, les cassettes peuvent être placées inclinées à un angle correspondant à l'angle d'inclinaison du front des cassettes (2).

4. Le système de lecture selon la revendication 1, **caractérisé en ce que** le capteur (4) peut lire tout le plateau (1) au moyen de prismes ou miroirs (5) angulaires.

5. Le système de lecture selon la revendication 1, **caractérisé en ce que** chacun des récipients (3) est un récipient à une rangée (3), le récipient à une rangée (3) peut être tourné en sens horaire et antihoraire de 90°, ce qui permet à trois côtés des cassettes (2) d'être visibles et lus par le capteur de lecture (4), et **en ce que** plusieurs récipients à une rangée (3) sont placés côte à côte à une distance telle qu'elle permet leur rotation horaire et antihoraire de 90° et sont maintenus ensemble par une courroie ou autre organe de transmission (6) qui garantit leur mouvement synchrone lors de la rotation.
